# EUROPEAN PATENT SPECIFICATION

(11) **EP 0 514 405 B1**
(45) Date of publication and mention of the grant of the patent: **04.01.1995**
(21) Application number: 91903044.5
(22) Date of filing: 18.01.1991
(51) Int. Cl.: C12N 15/70, C12N 1/20, C12R 1/19, C12N 15/71

(54) **STRAIN RAL-4, USEFUL FOR PRODUCTION OF SMALL PROTEINS AND PEPTIDES**
STAMM RAL-4, NÜTZLICH ZUR HERSTELLUNG KLEINER PROTEINE UND PEPTIDE
SOUCHE RAL-4- UTILE POUR LA PRODUCTION DE PROTEINES ET DE PEPTIDES DE FAIBLE POIDS MOLECULAIRE

(30) Priority: 31.01.1990 US 472640
(43) Date of publication of application: 25.11.1992
(73) Proprietor: THE UPJOHN COMPANY, Kalamazoo, Michigan 49001 (US)
(72) Inventor: LEPLEY, Robert Alan, Kalamazoo, MI 49008 (US); OLSON, Eric, Robert, Kalamazoo, MI 49009 (US)
(74) Representative: Perry, Robert Edward
(86) International application number: US9100265
(87) International publication number: WO9111523

(56) References cited:
- EP-A- 30 727
- EP-A- 138 526
- EP-A- 197 797
- WO-A-87/07296
- WO-A-88/06186
- CHEMICAL ABSTRACT, vol. 104, no. 1, 1986, Columbus, Ohio, USA HSU J.H. et al.
- BIOLOGICAL ABSTRACTS, vol. 86, 1988 SOBOTKOWA, L.P. et al.
- AGRICULTURAL AND BIOLOGICAL CHEMISTRY, vol. 53, no. 11, November 1989, TOKYO JP pp. 2941-2948; ISHIWATA, K.I. et al.
- METHODS IN ENZYMOLOGY, vol. 101, 1983, pp. 155-164; NICHOLS, B.P. & YANOVSKY, C:

## Description

### FIELD OF THE INVENTION

This invention relates to new strain of Escherichia coli, designated RAL-4, which is a tryptophan auxotroph and is capable of enhanced monomeric expression of low molecular weight proteins and peptides when suitably transformed.

### BACKGROUND OF THE INVENTION

Recombinant microorganisms can serve as important tools for the expression of biologically important compounds, typically proteins. The expression of foreign proteins in prokaryotic organisms has provided a useful means whereby relatively large quantities of homogeneous protein product can be obtained for agricultural and biomedical research, clinical purposes, and commercial use. By far, the prokaryote most commonly employed in recombinant protein production is Escherichi coli (E. coli). While higher molecular weight proteins, products with molecular weights greater than 15 kilodaltons (kd), are routinely expressed, small proteins and peptides, are either expressed poorly or not at all. Typically this problem is circumvented through the use of fusion protein, T.L. Moks et al., "Expression of Human Insulin-Like Growth Factor I in Bacteria: Use of Optimized Gene Fusion Vectors to Facilitate Protein Purification", Biochem. 26: 5239-5244 (1987), or by construction of synthetic genes containing mutimeric copies of the protein monomer. However, use of these methods create problems in isolation, cleavage, and yield of the protein expressed. (For a detailed discussion see P.K. Ghosh and D.K. Biswas, "Production of Therapeutic Polypeptides Through Esherichia Coli: A Perspective", Hindustan Antibiotics Bulletin 30, pp. 1-30 (1988) and F.A.O. Martson, "The Purification of Eukaryotic Polypeptides Synthesized in Escherichia Coli", Biochem. J. 240: 1-12 (1986)).

A further difficulty encountered by investigators attempting to express small monomeric proteins in E. coli is the rate of degradation by cellular proteolytic enzymes. For example, a synthetic gene encoding the seventy amino acid, monomeric insulin-like growth factor I (IGF-I) under control of lambda P_{L} promoter was used by G. Buell et al., "Optimizing the Expression in E. Coli of a Synthetic Gene Encoding Somatomedin-C (IGF-I)", Nucl. Acids Res. 13:1923-1938. (1985), in an attempt to express the protein in E. coli. These investigators found expressed IGF-I protein to be present at only several hundred molecules per cell and attributed the poor expression level to instability of the IGF-I product. This hypothesis was substantiated when strains mutant with respect to the lon and htpR (now called rpoH) locus were induced and found to express IGF-I at levels 33 fold higher than wildtype cells. This observation was further exploited by E.Y. Wong et al., "Expression of Secreted Insulin-Like Growth Factor-1 in Escherichia Coli", Gene 68:193-203 (1988), who employed the signal sequences for lamB and ompF to direct the secretion of monomeric IGF-I into the periplasmic space and thereby minimized the effects of intracellular proteolysis on IGF-I accumulation. While both of these systems did effect the expression of IGF-I in E. coli, neither achieved levels of expression which met production criteria.

We have sought to identify strains of E. coli which are capable of high level production of small proteins and peptides without resorting to covalent fusion strategies or synthesizing genes containing multimeric copies of the protein in tandem repeat structure. A plausible means whereby such strains may be created is through the use of in vitro chemical mutagenic agents. Chemical mutagens are well known to those skilled in the art as powerful techniques for identifying cells with a desired, selected phenotype. Their advantage lies in the random nature of the mutagens formed and the large number of cells which can be exposed and selected in a single experiment.

One such mutagenic agent is ethyl methanesulfonate (EMS). EMS appears to favor the creation of O6-alkylguanine residues resulting in base pair mismatching and G-C to A-T base pair transitions. Although several pathways exist by which cells can effect the repair of such damage, G.C. Walker, "Mutagenesis and Inducible Responses to Deoxyribonucleic Acid Damage in Escherichia Coli", Microbiol. Rev. 48:60-93, (1984), EMS is a particularly effective mutagen because it does not efficiently induce the cell's adaptive repair system and once induced, ethylation damage is inefficiently repaired by enzymatic processes designed to address methylation damage. B. Sedgwick and T. Lindahl, "A Common Mechanism for Repair of 06-Methylguanine and 06-Ethylguanine in DNA", J. Mol. Biol. 154:169-175, (1982). Therefore, use of this agent coupled to an efficient screening process employing immunoblotting procedures highly specific for a selected protein make an effective system for the identification of mutants which possess phenotypes indicative of enhanced expression levels.

The instant invention overcomes the problems referred to above by providing strains of E. coli which are able to produce monomeric copies of a specified protein once they are transformed by standard recombinant techniques to encode a protein of interest.

### INFORMATION DISCLOSURE

G. Buell et al., "Optimizing the Expression in E. Coli of a Synthetic Gene Encoding Somatomedin-C (IGF-I)", Nucl. Acids Res. 13:1923-1938 (1985) (discussed above) does not suggest use of EMS as a technique for enhancing protein expression. In addition, IGF expression in G. Buell et al. is under the control of a phage-derived promoter and IGF is expressed poorly.

The technique employed by E.Y. Wong, "Expression of Secreted Insulin-Like Growth Factor-1 in Escherichia Coli", Gene 68:193-203 (1988), minimizes exposure of IGF to proteases by directing the protein into the periplasmic space. However, the reference does not suggest the means employed herein nor does the present invention require the use of the signal sequences disclosed in the reference.

T.L. Moks et al., "Expression of Human Insulin-Like Growth Factor I in Bacteria: Use of Optimized Gene Fusion Vectors to Facilitate Protein Purification", Biochem. 26:5239-5244 (1987) cited above, reported the expression of human IGF-I in E. coli. However, the present invention does not require the fusion vector employed therein.

Y. Saito et al., "Direct Expression of a Synthetic Somatomedin C Gene in Escherichia Coli by Use of a Two-Cistron System", J. Biochem. 101:1281-1288 (1987), reported the direct expression of a synthetic somatomedin-C gene in E. coli. However, the present invention is mono-cistronic with respect to the IGF-I expression plasmid.

Plasmid pSK4 is employed in the present invention and is described by P.S. Kaytes et al., in "High Level Expression of Human Renin in Escherichia Coli", J. Biotechnology 4:205-218, (1986).

M.K. Olson et al., "Enhancement of Heterologous Polypeptide Expression by Alterations in the Ribosome-Binding-Site Sequence", J. Biotechnology 9:179-190 (1989), describes the pTrp2 vector used in this invention.

The use of EMS as a mutagenic agent is well known by those skilled in the art. The present invention uses the procedure of J. Miller, Experiments in Molecular Genetics, Cold Spring Harbor Laboratory, Cold Spring Harbor, N.Y. (1972), modified as described below.

The methods employed in the present invention also involve standard laboratory techniques involving recombinant DNA experimentation and phage transduction. Such methods may be found in T. Maniatis, et al, "Molecular Cloning", Cold Spring Harbor Laboratory, (1982).

### SUMMARY OF THE INVENTION

This invention provides a non-naturally occurring strain of Escherichia coli, which is designated RAL-4, having accession number NRRL B-18562.

More particularly, this invention provides a form of RAL-4 encoding heterologous protein deficient in tryptophan.

Most particularly, the present invention provides a form of RAL-4 characterized by enhanced monomeric production of insulin-like growth factor-I (IGF-I) or growth hormone releasing factor (GRF).

This invention also provides a process for producing heterologous protein by generating an E. coli auxotroph for an amino acid not resident in the heterologous protein to be expressed.

A preferred embodiment of the process of this invention provides a means for producing heterologous protein comprising transforming an E. coli auxotroph with a vector encoding a suitable heterologous protein, growing the transformant under suitable conditions, inducing expression of the protein, and purifying the expressed protein. Any suitable heterologous protein may be employed, however the method is particularly useful for expression of proteins and peptides having a molecular weight of approximately fifteen (15) kilodaltons or less.

Together, RAL-4 and the process of this invention provide a basis of a system for enhanced production of heterologous protein, preferrably IGF-I and GRF. This system further comprises the use of either a transformed form of RAL-4 or other tryptophan auxotroph.

### DETAILED DESCRIPTION OF THE INVENTION

The present invention provides a new strain of Escherichia coli and a form of this new strain characterized by enhanced expression of proteins or peptides in monomeric form. This new strain has been designated RAL-4 and was deposited on 11 November, 1989, at the Northern Regional Research Laboratory (NRRL), U.S. Dept. of Agriculture, Peoria, IL, and assigned accession number B-18562.

In addition, the present invention also provides a process for producing heterologous protein. This method involves generating E. coli auxotrophs for an amino acid not resident in the heterologous protein to be expressed. Any suitable means by which an auxotroph may be generated may be used, however P1 phage transduction is preferred. The auxotroph is subsequently transformed with an expression vector encoding the protein, expression induced, and the protein purified. A suitably transformed RAL-4, a tryptophan auxotroph, is particularly useful when employed in this method. Alternatively, E. coli DU99B, rendered auxotrophic by P1 phage transduction, may also be employed in this method.

The terms below are used throughout this disclosure and claims and are defined as follows:
"Heterologous protein" refers to a protein the genotype of which is derived from an organism other than the host organism.

"Enhanced production" or "enhanced expression" means a level of protein expression, as measured by Southern blot analysis, which is in excess of the amount expressed by wild-type controls.

"Auxotrophic" means an inability to synthesize a particular amino acid. An "auxotroph" is an organism which is auxotrophic and is only able to grow if the medium is supplemented with the particular amino acid.

"NRRL" refers to the Northern Regional Research Laboratory, U.S. Dept. of Agriculture, Peoria, Illinois.

Unless stated otherwise, "IGF-I" is insulin-like growth factor I of non-specific origin and "GRF" is growth hormone releasing factor of non-specific origin. "Leu²⁷b-GRF" is bovine growth hormone releasing factor in which methionine at position 27 is replaced with leucine.

We produced RAL-4 by combining recombinant techniques with chemical mutagenesis as briefly described here and in more detail below. We began by isolating a gene coding for insulin-like growth factor (IGF-I) from a porcine hepatic cDNA library. Alternatively, a a cDNA is synthesized using published amino acid sequences for IGF from porcine or other species. IGF-I expression vectors are then constructed by enzyme restriction and recombinant engineering of the cDNA by addition of linkers containing restriction sites designed to facilitate expression of the vector assembly, and initiation and N-terminal codons. Any appropriate restriction site may be engineered into the linker. Selection of an appropriate N-terminal codon as well as the vector used in subsequent transformations is also within the ordinary skill of one in the art. This construct (pIGF105) is ligated into an appropriate vector. In the preferred embodiment we use a pBr-based vector, pSK4, which contains the E. coli tryptophan promoter and ribosomal binding site. This plasmid is designated pIGF105/Ptrp. pIGF105/Ptrp is then restricted and the fragment containing the 70 amino acid coding sequence for IGF-I and the tryptophan promoter is ligated into pSK4 and is designated pIGF70/Ptrp. Restriction of pIGF70/Ptrp in turn provides a ClaI/BamHI fragment (representing the 70 amino acid IGF-I coding region), which is isolated and ligated into a vector encoding the Trp promoter. Preferrably, we use pTrp2 in subsequent mutagenesis.

E. coli strain BST-1c (NRRL B-18303) is then transformed using pTrp2 and treated with EMS. Cells which survive are induced for expression of IGF-I. Those mutants showing enhanced expression of the heterologous protein are selected and designated RAL-4. The mutation to the host cell DNA is confirmed by plasmid curing. We have also demonstrated that the EMS-induced mutation occurs to the host cell and, further, that RAL-4 is a tryptophan auxotroph.

We have also demonstrated the versatility of RAL-4 by transforming the strain with a plasmid encoding another protein lacking tryptophan, bovine growth hormone releasing factor (bGRF). Protein expression in this recombinant also exceeded the wild-type controls. IGF-I and GRF and their uses are well known in the art.

We have generated additional auxotrophic strains of E. coli by P1 phage transduction as described in detail below. After transformation with an appropriate expression vector, such as pIGF70/Trp2, such auxotrophs are capable of expressing protein product at levels in excess of the wild type parent.

Although the various embodiments described here are directed to expression of proteins which are growth factors, it will be clear to those skilled in the art that expression of any protein lacking the amino acid tryptophan can be enhanced by the use of similar techniques and suitable vectors. An example of such a peptide is corticotropin releasing factor (CRF). This 41 amino acid peptide which effects the release of corticotropin from the anterior pituitary gland has been shown by Y. Furutani et al., Nature, 301:537-540 (1983), to be lacking tryptophan residues in its mature sequence.

While Example 3 describes enhanced protein expression via phage transduction of the tryptophan operon, the techniques disclosed here may be employed to increase expression of proteins lacking amino acids other than tryptophan when a corresponding operon is disrupted in the host cell.

It should also be apparent that the techniques of this invention can be readily adapted by conventional means for commercial and industrial production by those skilled in the art. For instance, production quantities of protein may be obtained by controlling the quantity of the amino acid which is available to the organism. Prototrophic growth is sustained by supplying the necessary amino acid until desired cell density is achieved. At the desired density, the source of the amino acid is removed thus inducing expression of the desired product. The product can then be easily removed and purified from the culture medium.

Finally, one skilled in the art will recognize that E. coli other than DU99b may be used to create an E. coli auxotroph. Other suitable strains include DH5, JM103, K12, RV308, W3110, HB101, MC1061, and SK107.

It is apparent that the foregoing description provides a means by which organisms may be easily and efficiently transformed to produce large quantities of heterologous protein, provided, however, the heterologous protein is lacking an amino acid for which the transformed organism is auxotrophic.

Charts A, B, and C illustrate a construction of the present invention. The following conventions are used to illustrate plasmids and DNA fragments:
(1) The single line figures represent both circular and linear double-stranded DNA.
(2) Asterisks (*) indicate that the molecule represented is circular. Lack of an asterisk indicates the molecule is linear.
(3) Endonuclease restriction sites are indicated above the line.
(4) Genes are indicated below the line.
(5) Distance between genes and restriction sites are not to scale. The charts show their respective positions only.

Those skilled in the art will recognize that many of the methods employed to produce RAL-4 involve standard laboratory techniques. Recombinant methods may be found in T. Maniatis et al., "Molecular Cloning", Cold Spring Harbor Laboratory, (1982); those involving EMS mutagenesis follow essentially the method of J. Miller, Experiments in Molecular Genetics, Cold Spring Harbor Laboratory, Cold Spring Harbor, N.Y. (1972), modified as described below. All chemicals and materials employed are readily available from public sources. Ethyl methanesulfonate, ampicillin, tetracycline, chlortetracycline and fumaric acid were obtained from SIGMA, St Louis, MO. Media used to support the growth of the strains was from DIFCO, Detroit, MI. All chemicals used in the generation of buffers were of reagent grade or better. Recombinant human IGF-I protein was purchased from IMCERA, Terre Haute, IN, for use as an analytical standard in SDS/PAGE and Western analysis.

E. coli strains BST-1c (lambda^{r},rpoH₁₁₂,zhg::Tn10) and DU99b (lambda^{r},rpoH₁₁₂) are available from the NRRL, accession numbers B-18303 and B-18590, respectively. Plasmid pSK4 is described by P.S. Kaytes et al., "High Level Expression of Human Renin in Escherichia Coli", J. Biotechnology 4:205-218, (1986), cited above. pUC vectors are available from commercial sources such as Pharmacia P/L, Inc., Piscataway, NJ USA. Other pBR and pURA vectors are also readily available from commercial sources.

The following examples present illustrative but non-limiting embodiments of the present invention.

### EXAMPLE 1

### PREPARATION OF RAL-4 AND EXPRESSION OF INSULIN-LIKE GROWTH FACTOR I

### A. Construction of BST-1c/[pIGF70/Trp2]

An insulin-like growth factor-I (IGF-I) complementary DNA (cDNA) is isolated from a porcine hepatic lambda gt11 cDNA library using standard techniques as described in T. Maniatis et al., "Molecular Cloning", Cold Spring Harbor Laboratory, New York (1982). The specific source of the IGF-I cDNA is not critical as cDNA from species other than porcine work equally well. Further, published amino acid sequence data for various species are available and one skilled in the art may use such data to synthesize corresponding cDNA. (See, for example: bovine - A. Honegger et al., "Insulin-like Growth Factors I and II in Fetal and Adult Bovine Serum", J. Biol. Chem., 261:569-575 (1986); human - M.L. Bayne et al., "Structural Analogs of Human Insulin-like Growth Factor I with Reduced Affinity for Serum Binding Proteins and the Type 2 Insulin-like Growth Factor Receptor", J. Biol. Chem., 263:6233-6239 (1988); and porcine - A.F. Tavakkol et al., "Porcine Insulin-like Growth Factor-I (pIGF-I): Complimentary Deoxyribonucleic Acid Cloning and Uterine Expression of Messenger Ribonucleic Acid Encoding Evolutionarily Conserved IGF-I Peptides", Mol. Endo. 2:674-681 (1988)). Vectors used for the expression of IGF-I are constructed in a two step process. First, referring to chart A, IGF-I cDNA is restricted using Ava II and BamH I to generate a 430 base pair (bp) fragment containing the entire porcine IGF-I region as well as 80 bp of 3' untranslated region (proIGF-I). The 5' end of proIGF-I is engineered to accept a short linker containing a Cla I site, as well as addition of the Met codon required for E. coli translational initiation, the IGF-I N-Terminal Glycine codon, and an Ava II site. This is ligated Cla I to BamH I into a pBR based vector, pSK4, containing the E. coli tryptophan promoter and ribosomal binding site as described by Kaytes, (1986). This IGF-I plasmid construct containing the 105 amino acid proIGF-I cDNA is designated pIGF105/Ptrp. The second step in this process, as shown in chart B, involves isolation of the EcoR I to Bgl I fragment of pIGF105/Ptrp containing the entire trptophan promoter region as well as porcine IGF-I coding sequence representing amino acids 1 through 69. The 3' end of this fragment is engineered to provide two translation stop codons, a Hind III site and finally a BamH I site downstream from the Ala codon. This IGF-I construct coding for the mature 70 amino acid form of the protein is ligated into the pSK4 vector EcoR I to BamH I and designated pIGF70/Ptrp. Correct sequence of vectors is verified using dideoxy sequencing, F.S. Sanger et al., "DNA Sequencing with Chain-Terminating Inhibitors", Proc. Natl. Acad. Sci. USA. 74:5463-5467 (1977). Restriction of these vectors at the 5' Cla I site and any of the 3' sites facilitated the construction of vectors containing the Trp promoter with variations in the ribosomal binding site region. M.K. Olson et al., "Enhancement of Heterologous Polypeptide Expression by Alterations in the Ribosome-Binding-Site Sequence", J. Biotechnology 9:179-190 (1989). The pIGF70/Trp2 vector used in the EMS mutagenesis experiment and subsequent characterizations is assembled by ligation of the Cla I / BamH I fragment of pIGF70/Ptrp downstream of the pTrp2 vector described by Olson, id.

pIGF70/Trp2 is used to transform E. coli strain BST-1c following a modified CaCl₂ procedure as described by D.A. Morrison, "Transformation and Preservation of Competent Bacterial Cells by Freezing", Methods Enzymol, 68:326-331 (1979). Presence of the plasmid is confirmed by antibiotic selection and restriction mapping. From a streaked plate grown at 37° C in the presence of 100ug/ml tryptophan, a single colony is picked and grown to mid log phase in LB supplemented with 0.1% glucose. This culture, BST-1c/[pIGF70/Trp2], is harvested and pelleted.

### B. Ethyl methanesulfonate (EMS) Mutagenesis

BST-1c/[pIGF70/Trp2] cells are resuspended in Tris buffered saline (TBS) and EMS at 80 ul/ml is added at 37° C with shaking following the procedure described by Miller (cited above). EMS is sparingly soluble in aqueous solutions so careful mixing at this point is critical to the mutagenesis. EMS is also a powerful mutagen so care in handling the samples is mandatory from this point on. After 30 minutes the reaction is quenched and the cells are pelleted, washed, and transferred to fresh culture plates.

### C. IGF-I Induction

Strains were induced to assess expression of porcine IGF-I by one of two methods. For pBR or pUC based vectors this involves growing E. coli strains overnight in LB/glucose/ampicillin and then diluting this saturated culture 1:100 into M9/glucose/1%CAA/amp for induction. While this method is effective for inducing IGF constructions based on pBR or pUC vectors, it is not satisfactory for inductions involving the pURA runaway vector. Induction experiments employing the pURA vector are initiated by selecting a single healthy colony from a streaked plated and inoculating fresh induction media with this colony. This inoculum is grown to the desired density in induction media, cells are harvested, and subsequently processed for SDS PAGE. SDS PAGE was performed using the method of U.K. Laemmli "Cleavage of Structural Proteins During the Assembly of the Head of Bacteriophage T4", (1972). The discontinuous gel system found to best resolve IGF-I from E. coli proteins consisted of a 15% separating gel and a 4% stacking gel. Immunological detection of IGF-I was carried out using rabbit polyclonal antiserum generated against human IGF-I. Western blots are executed essentially as described by H. Towbin et al., "Electrophoretic Transfer of Proteins from Polyacrylamide Gels to Nitrocellulose Sheets: Procedure and Some Applications", Proc. Natl Acad. Sci USA. 76:4350-4354 (1979) against 250 ng of IGF-I standard. Selection of 250 ng IGF-I as the standard for comparison is based on appropriateness of autoradiographic signal strength generated by this quantity of standard relative to that expected from 0.25 OD₅₅₀/lane of a culture containing 1ug IGF-I per OD₅₅₀.

### D. Plasmid Curing

Plasmid cured derivatives of BST-1c/[pIGF70/Trp2] are isolated by screening for ampicillin sensitivity. Cells are inoculated into ampicillin free LB media supplemented with 0.1% glucose and 100 ug/ml tryptophan. Cultures are outgrown to saturation and then inoculated into fresh ampicillin free media for a second cycle of growth. A saturated culture is diluted and plated onto Antibiotic II plates supplemented with 100ug/ml tryptophan at a density which permits single colony isolation. From these plates, colonies are picked onto Antibiotic II plates supplemented with 100ug/ml ampicillin and then ampicillin free plates. Colonies which fail to grow on the ampicillin plates are further evaluated for curing. Mutant strains which fail to cure using this method are outgrown as described above in ampicillin free media supplemented with 4 or 6 ug/ml rifampicin. After two cycles of growth to saturation, cultures are plated, and single colonies picked onto ampicillin and ampicillin free plates as described above. Again, colonies which fail to grow on ampicillin plates are retained for further analysis.

### E. Identification of RAL-4

The population of cells surviving exposure to EMS, designated EMS 80, are surveyed for IGF-I expressing mutants. From a total population of approximately 12x10³ clones plated, fourteen clones which stained immuno-positive for IGF-I expression were isolated and subcloned for further characterization. Upon induction, six colonies were assessed as producing sufficient immuno-reactive porcine IGF-I in excess of the wild-type host, BST-1c/[pIGF70/Ptrp2], to warrant further consideration. In order to establish whether the expression phenotype is conferred by a mutation in the plasmid or by a combination of host-plasmid mutations, each of the six mutants is grown and the pIGF70/Trp2 plasmid isolated. Simultaneously, experimentation is carried out to cure each of the hosts of plasmid. Of the six mutants selected, a total of four were successfully cured of plasmid, two spontaneously and two through the introduction of rifampicin at 4ug/ml (as described above). A factorial experiment is designed in which BST-1c are transformed with plasmid isolated from the six mutants and conversely, each of the four cured hosts were transformed with wild type pIGF70/Ptrp2. Inductions are carried out and the results suggested that none of the plasmids isolated from hosts exposed to EMS enhanced expression over the wild type plasmid. However, induction of mutant hosts carrying wild type plasmid revealed that one of the mutant hosts expressed IGF-I at levels well in excess of the wild type host-plasmid combination as well as the wild type host-corresponding mutant plasmid combination; this mutant host is designated RAL-4. Thus, the expression phenotype of RAL-4 is conferred by a host mutation and not a plasmid mutation. Other mutant hosts express intermediate levels of IGF-I relative to BST-1c and RAL-4 and were not further characterized.

### F. Confirmation of RAL-4 Phenotype Changes

Tryptophan auxotrophy is confirmed in RAL-4 by selective plating on M9/0.1% glucose plates in the presence and absence of 100ug/ml of l-tryptophan. RAL-4 in the absence of tryptophan show little, if any, growth as compared to wild type BST-1c. To alleviate tryptophan auxotrophy and restore prototrophic growth, RAL-4 is transduced to Trp⁺ by P1 phage transduction essentially as described by Miller (1972). Transduction to Trp⁺ restores prototrophic growth. A tetracycline sensitive derivative of RAL-4, RAL-4 (Tet^{s}), is isolated by the method of B.R. Bochner, et al, "Positive Selection for Loss of Tetracycline Resistance", J. Bacteriology 143:926-933 (1980). RAL-4 made Tet^{s} by fumaric acid treatment and Trp⁺ by P1 transduction was transformed with pIGF70/-T-Trp2 and induced for IGF-I expression as described in the above sections. As expected, in comparison to the auxotrophic RAL-4 parent, RAL-4 (Trp⁺, Tet^{s}) variant displayed growth characteristics which were superior to the RAL-4 (Trp^{-,} Tet^{r}) counterpart and similar to the prototrophic growth of the BST-1c wildtype. Induction data indicate that the Trp⁺ RAL-4 variants do not express IGF-I at levels comparable to the Trp⁻ strains and moreover, IGF-I expression was barely detectable except at very heavy gel loads. While these data imply a correlation between tryptophan biosynthesis and IGF-I expression, alternative explanations such as a slow outgrowth requirement (displayed by the trp⁻ RAL-4 variant) for expression are also possible. However, these results do indicate that RAL-4 is a tryptophan auxotroph, that the auxotrophy is required for IGF-I expression, but the phenotype does not require tetracycline resistance.

### EXAMPLE 2

### RAL-4 EXPRESSION OF GROWTH HORMONE RELEASING FACTOR

A plasmid containing a synthetic bovine growth hormone releasing factor (bGRF) is transformed into RAL-4. This plasmid, pBGRF1, is a pBR322-based expression vector encoding bGRF in which the methionine at position 27 is substituted by leucine ((Leu²⁷)bGRF). This analog has been shown by T. Kempe, et al, Bio/Technology 4, 565-568 (1986), to have full biological activity. In addition to bovine, growth hormone releasing factors from other species are within the scope of this invention. Sequences for GRFs from various species have been compiled by N. Ling et al., "Growth Hormone Releasing Factors", Ann. Rev. Biochem. 54:403-423 (1985). The bovine oligonucleotide is synthesized on an Applied BioSystems 380B DNA synthesizer. After deprotection, the oligonucleotide is purified by polyacrylamide gel electrophoresis under denaturing conditions followed by desalting with Waters Sep-Pak C-18 cartridges according to N.Y. Theriault, et al, Nucleosides and Nucleotides 5, 15-32 (1986). The oligonucleotide is ligated with T4 DNA ligase according to the protocol of N.Y. Theriault et al., Biotechniques 6, 470-474 (1988). The ligated product is purified by 12% polyacrylamide gel electrophoresis under non-denaturing conditions and isolated by electroelution.

The synthetic (Leu²⁷)bGRF gene, as well as initiation and translation codons, are cloned into the ClaI-HindIII region of pTrp (see Chart C), which places the gene downstream of the ConSD variant of the E. coli tryptophan promoter described by M.K. Olson et al., "Enhancement of Heterologous Polypeptide Expression by Alterations in the Ribosome-Binding-Site Sequence", J. Biotechnology 9:179-190 (1989). Induction followed by SDS-PAGE and Western blotting confirms that RAL-4 expresses immunoreactive bGRF peptide at levels in excess of BST-1c wild type cells transformed with the same plasmid. Thus the mutation present in RAL-4 is not allele specific and is capable of increasing expression of a smaller peptide. The expressed bGRF sequence reveals that this 5 kd protein does not contain tryptophan. Thus a primary criteria for enhanced expression in RAL-4 resides in tryptophan deletion from the heterologous protein itself.

### EXAMPLE 3

### GENERATION OF RAL-4 EXPRESSION PHENOTYPE BY PHAGE TRANSDUCTION

Tryptophan auxotrophy was generated in E. coli strain DU99b by P1 phage transduction of a Tn10 transposon into the tryptophan B locus of the tryptophan operon. The product of this structural gene is the beta subunit of tryptophan synthase (EC 4.2.1.20) an enzyme required for tryptophan biosynthesis. The genetic source for the trpB::Tn10 transposon was E. coli strain EC-O described by Chakrabarti, S. L. and L. Gorini, Proc. Natl. Acad. Sci. 2084-2087 (1972). The methodology employed in the P1 phage transductions has been described by J. Miller, "Experiments in Molecular Genetics", Cold Spring Harbor Laboratory, Cold Spring Harbor, N.Y. (1972) and is presented in greater detail below.

E. coli strain EC-O (trpB::Tn10) used to transduce E. coli strain DU99b to tryptophan auxotrophy was grown to saturation in LB supplemented with 0.1% glucose and 15ug/ml tetracycline. A 100ul volume of this culture was removed and added to 10 ml of LB medium supplemented with 5 mM CaCl2. To this was added 1ul of P1 phage and the EC-O/P1 mixture incubated at 37° C with shaking at 200 RPM until lysis was evident. A 100ul volume of chloroform was added and the mixture held at 37° C for an additional 10 minutes. Cellular debris were removed by centrifugation at 10,000 x Gₐᵥₑ, the supernatant recovered and again, supplemented with 100ul of chloroform and stored at 4° C as a P1 phage stock.

The transduction of E.coli strain DU99b was carried out using standard techniques. Briefly, DU99b cells were grown to saturation in LB media supplemented with 0.1% glucose. From this saturated culture 1ml was removed and the cells pelleted at 8000 x Gₐᵥₑ for 10 minutes at 4° C. The cellular pellet was resuspended in 0.5 ml of 10 mM MgSO₄, 5 mM CaCl₂. To this suspension was added 250ul of the EC-O P1 phage stock and the resulting mixture incubated at 30° C with gentle shaking at 150 RPM for 30 minutes. Subsequent to this incubation, 0.5 ml of 1M sodium citrate was added and the cells pelleted as described above. The resulting cellular pellet was resuspended in 0.5 ml of 1M sodium citrate and 1 ml of LB media supplemented with 0.1 % glucose. This mixture was incubated at 37° C for 1 hour to permit the development of tetracycline resistance and the cells plated on ABII/agar plates supplemented with 15ug/ml tetracycline. From the population of cell producing colonies, 15 were selected and purified on ABII/agar supplemented with 15ug/ml tetracycline. Purified cells were streaked onto M9 minimal salts agar plates supplemented with 15ug/ml tetracycline to verify the presence of tryptophan auxotrophy.

DU99b cells established to be genotypically trpB::Tn10 and phenotypically tryptophan auxotrophs were transformed with the pIGF70/Trp2 expression vector. Induction followed by SDS-PAGE followed by Western analysis confirmed that the DU99b tryptophan auxotroph was capable of expressing immunoreactive IGF-I at levels in excess of those of DU99b control cells and comparable to those of the RAL-4 mutant. Thus tryptophan auxotrophy created by P1 phage transduction within the trpB gene is capable of creating the RAL-4 expression phenotype. Furthermore it should be apparent that the creation of tryptophan auxotrophy by inactivation of any of the structural genes of the tryptophan operon would be sufficient to create the RAL-4 expression phenotype.

### CHART A. Construction of pIGF105

(a) IGF-I cDNA is digested with AvaII and BamHI to yield proIGF-I. 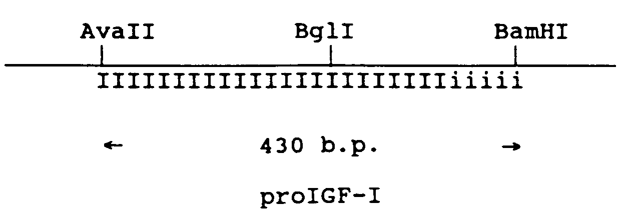
AvaII BglI BamHI


IIIIIIIIIIIIIIIIIIIIIIIIIIIIIiiiii

← 430 b.p. →

proIGF-I
(b) proIGF-I is engineered to include ClaI and AvaII restriction sites and a Met codon. 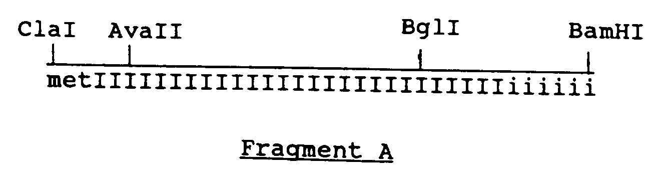
ClaI AvaII BglI BamHI
metIIIIIIIIIIIIIIIIIIIIIIIIIIIIIiiiiii

Fragment A
(c) Fragment A is ligated into pSK4 to produce pIGF105/Ptrp.

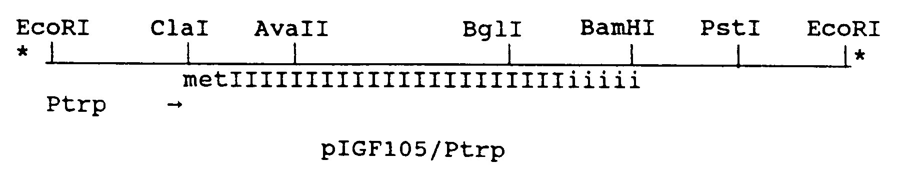
EcoRI    ClaI    AvaII    BglI    BamHI    PstI    EcoRI
*                                                    *
metIIIIIIIIIIIIIIIIIIIIIIIiiiii
Ptrp    →

pIGF105/Ptrp

### CHART B. Construction of pIGF70/Ptrp.

(a) pIGF105/Ptrp is digested with EcoRI and BglI, and engineered to include BamHI and HindIII, to form pIGF70. 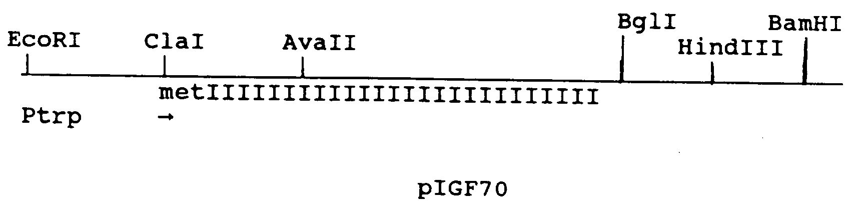
EcoRI ClaI AvaII BglI HindIII BamHI
metIIIIIIIIIIIIIIIIIIIIIIIII
Ptrp →
pIGF70
(b) pIGF70 is ligated into pSK4 to form pIGF70/Ptrp.

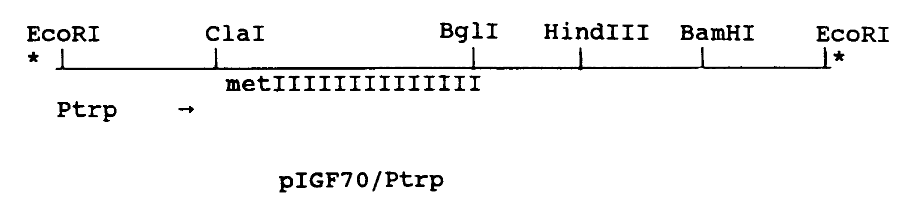
EcoRI ClaI BglI HindIII BamHI EcoRI
* *
metIIIIIIIIIIIII
Ptrp →

pIGF70/Ptrp

### CHART C. Construction of pBGRF1

(a) (Leu²⁷)bGRF is engineered to include flanking methionine codons, BglII and BamHI sites, initiation codons, a 5' ClaI site, and a 3' HindIII site. 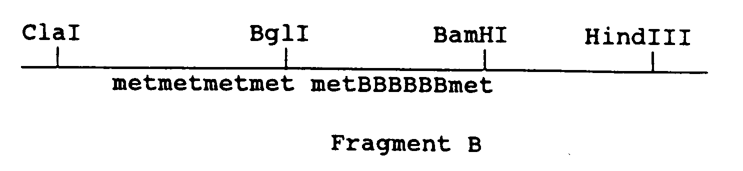
ClaI BglI BamHI HindIII
metmetmetmet metBBBBBBmet

Fragment B
b) pTrp is digested with ClaI and HindIII and Fragment B ligated ClaI-HindIII.

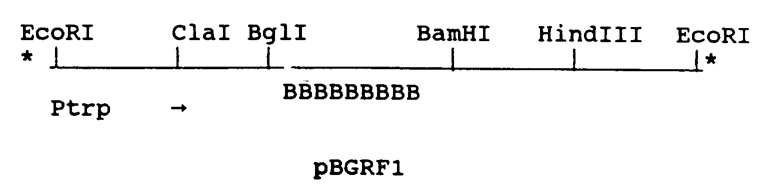
EcoRI ClaI BglI BamHI HindIII EcoRI
* *
BBBBBBBBB
Ptrp →
pBGRF1

## Claims

1. The strain of E. coli, which is designated RAL-4, having accession number NRRL B-18562.

2. E. coli of claim 1, further comprising nucleotide bases encoding heterologous protein deficient in tryptophan.

3. E. coli of claim 2, wherein the heterologous protein has a molecular weight of approximately fifteen kilodaltons or less.

4. E. coli of claim 3, wherein the heterologous protein is IGF-I.

5. E. coli of claim 3, wherein the heterologous protein is porcine IGF-I.

6. E. coli of claim 3, wherein the heterologous protein is GRF.

7. E. coli of claim 3, wherein the heterologous protein is Leu²⁷bGRF.

8. A process for producing heterologous protein, which comprises:
(a) transforming an E. coli auxotroph with a vector encoding the heterologous protein, said protein lacking the amino-acid for which the E. coli is auxotrophic;
(b) growing the transformant under suitable conditions;
(c) inducing expression of the protein; and
(d) purifying the expressed protein.

9. A process according to claim 8, wherein the auxotroph is RAL-4, having accession number NRRL B-18562.

10. A process according to claim 8, which additionally comprises generating the auxotroph by P1 phage transduction.

11. A process according to any of claims 8 to 10, wherein the protein is as defined in any of claims 3 to 7.

## Patentansprüche

1. E. coli Stamm, der als RAL-4 bezeichnet wird, mit der Hinterlegungsnummer NRRL B-18562.

2. E. coli nach Anspruch 1, des weiteren umfassend Nucleotidbasen mit Codierung für ein heterologes Protein, dem Tryptophan fehlt.

3. E. coli nach Anspruch 2, wobei das heterologe Protein ein Molekulargewicht von etwa 15 Kilodalton oder weniger aufweist.

4. E. coli nach Anspruch 3, wobei das heterologe Protein IGF-I ist.

5. E. coli nach Anspruch 3, wobei das heterologe Protein Schweine IGF-I ist.

6. E. coli nach Anspruch 3, wobei das heterologe Protein GRF ist.

7. E. coli nach Anspruch 3, wobei das heterologe Protein Leu²⁷bGRF ist.

8. Verfahren zur Herstellung eines heterologen Proteins, umfassend
(a) Transformieren eines auxotrophen E. coli Organismus mit einem Vektor mit Codierung für das heterologe Protein, wobei dem Protein die Aminosäure fehlt, für die der E. coli auxotroph ist,
(b) Wachsenlassen des Transformanten unter geeigneten Bedingungen,
(c) Induzieren einer Expression des Proteins und
(d) Reinigen des exprimierten Proteins.

9. Verfahren nach Anspruch 8, wobei der auxotrophe Organismus aus RAL-4 mit der Hinterlegungsnummer NRRL B-18562 besteht.

10. Verfahren nach Anspruch 8, zusätzlich umfassend eine Erzeugung des auxotrophen Organismus durch P1 Phagentransduktion.

11. Verfahren nach einem der Ansprüche 8 bis 10, wobei das Protein der Definition nach einem der Ansprüche 3 bis 7 genügt.

## Revendications

1. Souche de E. coli, qui est appelée RAL-4, à laquelle est attribué le numéro de dépôt NRRL B-18562.

2. E. coli suivant la revendication 1, comprenant en outre les bases nucléotidiques codant pour une protéine hétérologue déficiente en tryptophane.

3. E. coli suivant la revendication 2, dans laquelle la protéine hétérologue possède un poids moléculaire égal ou inférieur à approximativement quinze kilodaltons.

4. E. coli suivant la revendication 3, dans laquelle la protéine hétérologue est le IGF-I.

5. E. coli suivant la revendication 3, dans laquelle la protéine hétérologue est le IGF-I porcin.

6. E. coli suivant la revendication 3, dans laquelle la protéine hétérologue est le GRF.

7. E. coli suivant la revendication 3, dans laquelle la protéine hétérologue est le Leu²⁷bGRF.

8. Procédé de production d'une protéine hétérologue, qui comprend :
(a) la transformation d'une souche auxotrophe de E. coli avec un vecteur codant pour la protéine hétérologue, ladite protéine étant dépourvue de l'amino-acide pour lequel la souche de E. coli est auxotrophe ;
(b) la culture du transformant dans des conditions convenables ;
(c) l'induction de l'expression de la protéine ; et
(d) la purification de la protéine exprimée.

9. Procédé suivant la revendication 8, dans lequel la souche auxotrophe est RAL-4, à laquelle a été attribué le numéro de dépôt NRRL B-18562.

10. Procédé suivant la revendication 8, qui comprend en outre la production de la souche auxotrophe par transduction avec le phage P1.

11. Procédé suivant l'une quelconque des revendications 8 à 10, dans lequel la protéine répond aux définitions suivant l'une quelconque des revendications 3 à 7.
